# EUROPÄISCHE PATENTSCHRIFT

(11) **EP 0 697 200 B1**
(45) Veröffentlichungstag und Bekanntmachung des Hinweises auf die Patenterteilung: **05.01.2000**
(21) Anmeldenummer: 95102545.1
(22) Anmeldetag: 23.02.1995
(51) Int. Cl.: A61F 2/44

(54) **Ventrales Zwischenwirbelimplantat**
Ventral intervertebral implant
Implant intervertébral ventral

(30) Priorität: 20.08.1994 DE 9413471 U
(43) Veröffentlichungstag der Anmeldung: 21.02.1996
(73) Patentinhaber: SCHÄFER micomed GmbH, 73035 Göppingen (DE)
(72) Erfinder: Schäfer, Bernd, D-73614 Schorndorf (DE); Schmitz, Stephan, D-73164 Schorndorf (DE)
(74) Vertreter: Dreiss, Fuhlendorf, Steimle & Becker, Patentanwälte

(56) Entgegenhaltungen:
- EP-A- 0 260 044
- WO-A-90/00037
- DE-C- 4 327 054
- US-A- 4 599 086

## Beschreibung

Die Erfindung betrifft ein ventrales Zwischenwirbelimplantat zum Einsatz beispielsweise zwischen zwei Wirbelkörper einer Wirbelsäule mit einer oberen und einer unteren Anlagefläche und mindestens einem wenigstens eine der Anlageflächen überragenden Ankerbolzen, wobei der mindestens eine Ankerbolzen versenkbar angeordnet ist und das Implantat zum Ausfahren des Ankerbolzens ein Getriebe aufweist, gemäß dem Oberbegriff des Anspruchs 1.

Die einzelnen Wirbel der Wirbelsäule weisen unter anderem einen Wirbelkörper, einen Wirbelbogen, einen Dornfortsatz, zwei Querfortsätze und zwei obere und zwei untere Gelenkfortsätze auf. Die Wirbel sind über an ihren Wirbelkörpern (Corpus vertebrae) anliegenden Zwischenwirbelscheiben (Disci intervertebralis) miteinander verbunden. Diese Zwischenwirbelscheiben bestehen aus flüssigkeitsreichem Faserknorpel und verbinden die einzelnen Wirbelkörper miteinander. Die Größe der Zwischenwirbelscheiben nimmt von oben nach unten entsprechend der im menschlichen Körper auftretenden Belastung zu. Die Zwischenwirbelscheiben dienen als elastische Puffer und dämpfen federnd Stöße ab. Es ist bekannt, daß sich die Zwischenwirbelscheiben verlagern können oder daß der innere Gallertkern (Nucleus pulposus) durch Risse im bindegewebartigen knorpeligen äußeren Ring (Annulus fibrosus) austreten kann. Dabei kann die Zwischenwirbelscheibe teilweise in die Zwischenwirbellöcher (Foramina intervertebralia) bzw. in den Spinalkanal eintreten. Außerdem kann dieser Prolaps medial bzw. dorsalmedial oder lateral sein. Derartige Prolapse treten am häufigsten an den L₄-L₅, L₅-S₁ und C₆-C₇-Vertebrales auf. Werden derartige Prolapse nicht therapiert kommt es zu irreversiblen Druckschädigungen von Nervenwurzeln oder zu Querschnittsläsionen. Sollte eine symtomatische Physiotherapie, z.B. Krankengymnastik oder Massage, keinen Erfolg versprechen, muß die Discus intervertebralis operativ entfernt werden. Nun besteht die Möglichkeit der Implantation einer künstlichen Zwischenwirbelscheibe oder der Osteosynthese der beiden Wirbel über ein starres Zwischenwirbelimplantat.

Aus der EP 392 076 A1 ist eine künstliche Zwischenwirbelscheibe bekannt geworden, die aus einer oberen und unteren Anlagefläche und einer elastischen Zwischenschicht besteht. Aus der Anlagefläche ragen Ankerbolzen heraus, über die die künstliche Zwischenwirbelscheibe an den Wirbelkörpern fixiert wird. Als nachteilig hat sich herausgestellt, daß diese bekannte künstliche Zwischenwirbelscheibe nur mit Mühe zwischen die beiden Wirbelkörper einsetzbar ist, da die Ankerbolzen das Einsetzen behindern.

Aus der US 5,192,327 ist ein starres Zwischenwirbelimplantat bekannt geworden, welches zur Osteosynthese ebenfalls zwischen zwei Wirbelkörper eingesetzt wird. Die beiden Anlageflächen dieses Implantats sind mit mit V-förmigen Längsnuten versehen, wodurch die Fixierung dieses Implantats an den Wirbelkörpern erzielt werden soll. Es hat sich gezeigt, daß insbesondere in den ersten zwölf bis sechszehn Wochen nach der Implantation das Implantat besonders leicht verrutscht, da zu diesem Zeitpunkt die Fixierung über die V-förmigen Nuten nicht ausreichend ist. Erst allmählich wächst Bindegewebe in die Durchbrüche des Implantats ein und fixiert so das Implantat am Wirbelkörper.

Mit der WO 90/00037 ist eine künstliche Zwischenwirbelscheibe bekannt geworden, die die Anlagefläche überragende Ankerbolzen aufweist. Bei dieser Zwischenwirbelscheibe müssen aber die einzelnen Ankerbolzen schwenkbar gelagert sein, wobei der Schwenkarm eine gewisse Länge aufweisen muß. Im Bereich dieses Schwenkarmes können keine Ankerbolzen vorgesehen werden. Die Anzahl der Ankerbolzen ist daher auf eine gerine Zahl beschränkt. Außerdem beschreiben die Ankerbolzen beim Ausfahren einen Kreisbogen, was unter Umständen unerwünscht ist. Eine optimale Fixierung ist daher nicht immer gewährleistet.

Mit der US-A-4,599,086 ist ein Zwischenwirbelimplantat bekannt geworden, bei dem das Getriebe von einem aus dem Implantat entfernbaren Zahnrad und von an den Ankerbolzen vorgesehenen Zähnen gebildet wird. Nach dem Ausfahren der Ankerbolzen ist das Zahnrad aus dem Implantat entfernbar.

Der Erfindung liegt daher die Aufgabe zugrunde, ein Zwischenwirbelimplantat mit dem eingangs genannten Aufbau bereitzustellen, welches einerseits einfach z.B. ventral oder dorsal zwischen die Wirbelkörper einzuführen und optimal an diesen fixierbar ist und außerdem zur Kostendämpfung beiträgt.

Diese Aufgabe wird durch ein Zwischenwirbelimplantat gelöst, das die Merkmale des Anspruchs 1 aufweist.

Durch die versenkbare Anordnung des bzw. der Ankerbolzen kann somit das Implantat bei versenktem bzw. versenkten Ankerbolzen problemlos zwischen die beiden Wirbelkörper eingeschoben werden, d.h. der Abstand zwischen den beiden Wirbelkörpern muß nicht größer sein als die Höhe des Implantats. Neben der einfacheren Plazierung müssen die beiden Wirbelkörper nach dem Entfernen der Zwischenwirbelscheibe zum Einführen des Implantats nicht noch weiter auseinandergedrängt werden, was unter Umständen Verletzungen an den Gelenkflächen zur Folge haben könnte. Der Abstand der Wirbelkörper vor und nach der Operation ist in der Regel gleich bzw. entspricht dem Abstand als ob eine gesunde Zwischenwirbelscheibe vorhanden wäre.

Um nach dem Plazieren des Implantats dieses mit den Wirbelkörpern zu fixieren, werden die Ankerbolzen über das Getriebe aus der versenkten Lage ausgeschoben und dringen in die Wirbelkörper ein. Hierdurch wird eine optimale Primärfixierung des Implantats erreicht, so daß die Ruhigstellung des Patienten bereits kurze Zeit nach der Operation aufgehoben werden kann. Die Primärfixierung durch die ausgefahrenen Ankerbolzen hat außerdem den Vorteil, daß das Implantat und die anliegenden Wirbelkörper keine Relativbewegungen zueinander ausführen können und dadurch das Bindegewebe sofort nach der Operation in das Implantat eindringen kann. Hierdurch wird der Heilprozeß wesentlich beschleunigt.

Das Getriebe ist als Instrument ausgebildet und wird nach dem Ausschieben der Ankerbolzen in deren Arbeitslage, in der sie die Oberfläche(n) des Implantats überragen, aus dem Implantat entfernt. Dies hat den entscheidenden Vorteil, daß das Getriebe mehrmals verwendbar ist, was erheblich zur Kostendämpfung beiträgt. Derartige Getriebe sind, wie weiter unten noch ausführlicher erläutert, zum Teil sehr aufwendig in der Herstellung, weshalb eine nur einmalige Nutzung nicht gerechtfertigt ist.

Bei einem Ausführungsbeispiel weist das Getriebe eine axiale Gewindebohrung auf, in die ein Gewindezapfen einer manuell betätigbaren Instrumentenstange eindrehbar ist.

Vorteilhaft verrasten die Ankerbolzen in ihrer Arbeitslage nachdem sie über das Getriebe ausgeschoben worden sind. Bei einer Weiterbildung ist vorgesehen, daß diese Verrastung wieder lösbar ist. Außerdem können die Ankerbolzen über das Getriebe wieder eingefahren werden. Das Getriebe ist dorsal oder ventral in das Implantat einsetzbar. Hierfür weist das Implantat entsprechende Öffnungen bzw. eine Durchgangsöffnung auf.

Das Getriebe weist eine drehbar gelagerte, den Ankerbolzen ausschiebende Nocke auf. Bei diesem Getriebe werden Bewegungs- und Kraftrichtungen in Richtung des Ankerbolzens umgelenkt. Dabei kann z.B. durch eine Verschiebe- oder Drehbewegung der Ankerbolzen aus seiner versenkten Lage durch entsprechende Ausnehmungen in der Anlagefläche hindurch ausgeschoben und in den Wirbelkörper eingeschoben werden. Dabei sind mehrere Ankerbolzen über eine oder mehrere Nocken ausschiebbar. Auf diese Weise können gleichzeitig mehrere Ankerbolzen aus ihrer Ruhelage in die Arbeitslage gebracht werden und in einen oder beide Wirbelkörper eindringen.

Die eine Nocke oder mehrere Nocken sind auf einer oder mehreren Wellen angeordnet. Insbesondere bei größeren Implantaten, z.B. für die Vertebrae lumbales des Lendenwirbelbereiches empfiehlt es sich, wenigstens in zwei Ebenen Ankerbolzen anzuordnen, die über zwei Wellen betätigt werden. Für die Vertebrae cervicales des Halswirbelbereiches, d.h. für den Bereich der C₁-C₇-Wirbeln genügt eine Welle, über die die Ankerbolzen in einer Ebene ausgeschoben werden.

Bevorzugt ist die Welle in einer zwischen den Anlageflächen vorgesehenen Bohrung gelagert. Derartige Lagerungen sind einfach herzustellen und nehmen die gesamte Welle vollständig auf. Dabei kann die Bohrung derart angeordnet sein, daß die Welle ventral oder dorsal oder lateral zugänglich ist.

Eine einfache Handhabung der Welle wird dadurch erzielt, daß sie über ein Werkzeug bewegbar ist und ein Innengewinde oder eine entsprechende Werkzeugangriffseinrichtung aufweist. Ein Innengewinde wird bevorzugt, da mit diesem die Nockenwelle auf einfache Weise mit dem Werkzeug verbindbar ist, bzw. verschiedene Wellen mit dem Werkzeug verbindbar sind.

Erfindungsgemäß ist die Welle nach Art einer Nockenwelle mit einer oder mehreren axial hintereinander angeordneten Nocken ausgebildet. Wird eine derartige Welle um ihre Achse gedreht, dann werden die Ankerbolzen über die Nocken gleichzeitig oder nacheinander ausgeschoben. Ein gleichzeitiges Ausschieben hat den Vorteil, daß die Nocken mit einem sehr langen Stellweg versehen werden können, wohingegen ein sequenzieller Ausschub den Vorteil hat, daß gezielt nur die erforderliche Anzahl von Ankerbolzen ausgeschoben werden kann.

Bevorzugt ist zwischen den Nocken oder zwischen mehreren Nocken jeweils ein die Nocken radial überragender, scheibenförmiger Bund vorgesehen. Da die Achsbolzen mit ihrer proximalen Stirnseite auf der Nocke aufliegen, wird ein Auszug der Welle aus der Bohrung über die radial überstehenden Bünde verhindert, da diese seitlich an den Ankerbolzen anliegen.

Dabei bildet vorteilhaft ein jeder Bund eine Lagerstelle für die Welle. Entsprechend kann die Oberfläche eines jeden Bundes so aufbereitet sein, daß entweder eine geringe Reibung für eine leichte Verstellung bzw. Verdrehung der Welle oder eine hohe Reibung als Sicherheit gegen ein selbsttätiges Verdrehen der Welle erzielt wird.

Bei einem Ausführungsbeispiel erstreckt sich der Stellweg der Nocke über 180° oder 360°. Bei einem Stellweg von 360° verteilt sich die gesamte Stellkraft auf eine volle Umdrehung der Welle. Bei einem Stellweg von 180° können auf gegenüberliegenden Seiten Ankerbolzen vorgesehen sein, die dann gleichzeitig durch eine halbe Umdrehung der Welle ausgefahren werden können. Dabei kann ein Nocken mit zwei sich über jeweils 180° erstreckende Stellwege versehen sein.

Falls die Welle nur eine Drehrichtung aufweist, kann die Welle mit einem Drehanschlag versehen sein. Durch diesen Anschlag werden Fehlbedienungen vermieden, insbesondere ein Überdrehen der Welle verhindert.

Gemäß einem bevorzugten Ausführungsbeispiel weisen die Ankerbolzen einen zylindrischen Querschnitt auf und sind in entsprechenden Ausnehmungen im Implantat gelagert. Die Ankerbolzen können z.B. als kreiszylindrische Stifte mit distaler Spitze ausgebildet sein, die mit ihrem proximalen flachen Ende auf der Nocke aufliegen.

Eine optimale Anpassung des Implantats an dei Form von Zwischenwirbelscheiben wird dann dadurch erzielt, daß das Implantat keilförmig ausgebildet ist und die beiden Anlageflächen einen Keilwinkel von 2° bis 15°, insbesondere von 3° bis 10° einschließen. Vor allem im Bereich der C₁-C₇-Wirbel, die eine Lordosestellung einnehmen, wird die Lage der miteinander zu verbindenden Wirbel über das Implantat beibehalten. Entsprechendes gilt für die Vertebrae lumbales, wo die Wirbel ebenfalls eine Lordosestellung einnehmen. Bei der Vertrebae thoracales nehmen die einzelnen Wirbel eine Kyphosestellung ein, so daß hier Implantate mit einem um 180° gedrehten Keilwinkel eingesetzt werden können.

Auch die Dicke des Implantats entspricht dem jeweiligen Einsatzort, d.h. bei der Vertebrae cervicales werden dünnere Implantate eingesetzt als bei der Vertebrae lumbales. Zusätzlich zur Primärfixation weist das Implantat zur Langzeitfixation wenigstens eine Anlagefläche mit einer profilierten Oberfläche auf, die insbesondere mit einer Sägeverzahnung versehen ist. Durch die Sägeverzahnung kann das Implantat gezielt gegen einseitig wirkende Verschiebekräfte gesichert werden. Vorteilhaft weisen beide Anlageflächen Sägeverzahnungen auf, die in verschiedene Richtungen, z.B. die eine ventral und die andere dorsal hemmen. Derart ausgebildete Implantate sind besonders unanfällig gegen Verschiebekräfte z.B. gegen ein weiteres Einschieben in dorsaler Richtung bzw. gegen ein Ausschieben in ventraler Richtung.

Weitere Vorteile, Merkmale und Einzelheiten ergeben sich aus der nachfolgenden Beschreibung, in der unter Bezugnahme auf die Zeichnung besonders bevorzugte Ausführungsbeispiele im einzelnen dargestellt sind. In der Zeichnung zeigen:
- Figur 1: eine Draufsicht auf ein ventrales Zwischenwirbelimplantat;
- Figur 2: eine Seitenansicht des Zwischenwirbelimplantats der Figur 1;
- Figur 3: einen Schnitt III-III gemäß Figur 2;
- Figur 3a: einen Schnitt durch eine Halteeinrichtung für die Ankerbolzen;
- Figur 4: einen Schnitt IV-IV gemäß Figur 3 mit versenkt angeordneten Ankerbolzen;
- Figur 5: einen Schnitt IV-IV gemäß Figur 3 mit ausgefahrenen Ankerbolzen;
- Figur 6: eine Stirnansicht der Nockenwelle der Figur 4;
- Figur 7: einen Schnitt VII-VII gemäß Figur 5 durch die Nockenwelle;
- Figur 8: einen Querschnitt durch eine weitere Ausführungsform einer Nockenwelle;
- Figur 9: einen Querschnitt durch eine dritte Ausführungsform einer Nockenwelle; und
- Figur 10: eine schematische Darstellung eines Vertebrae thoracicae mit aufliegendem Zwischenwirbelimplantat.

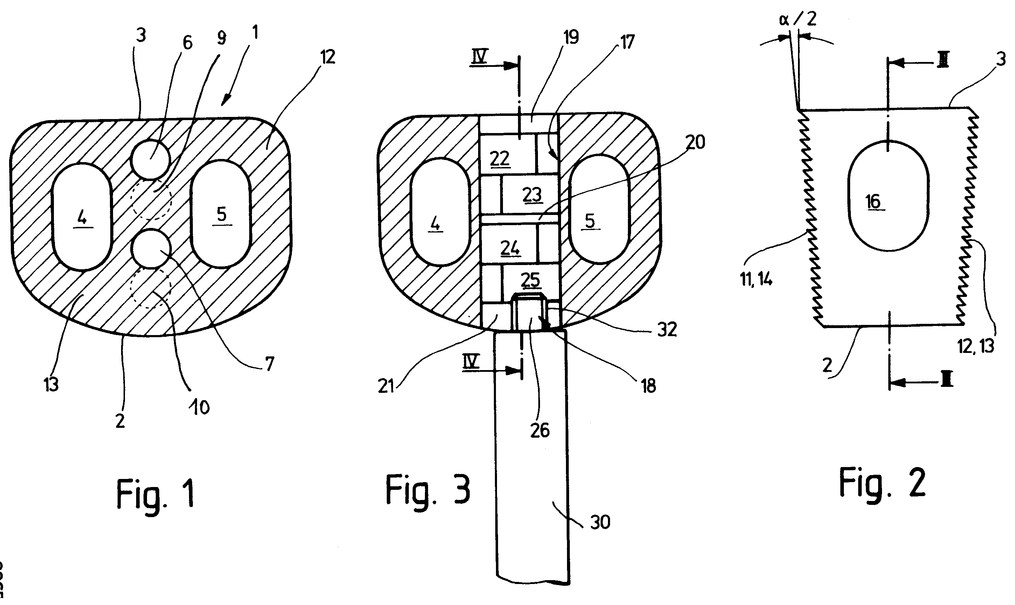

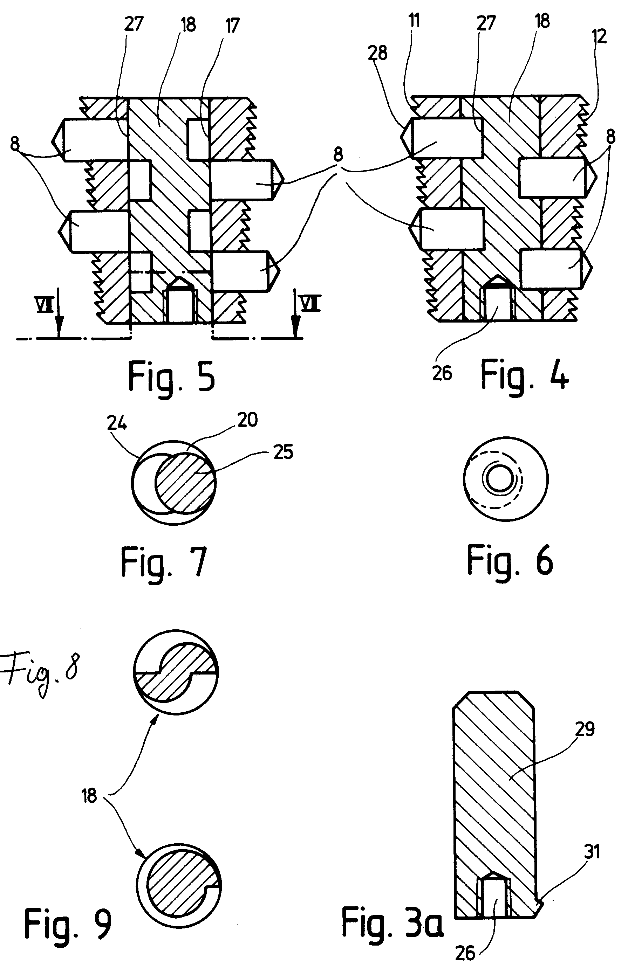

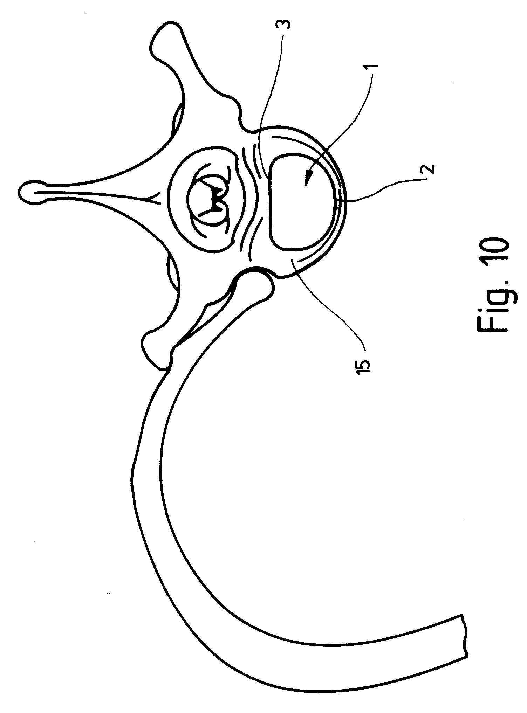

Die Figur 1 zeigt ein insgesamt mit 1 bezeichnetes ventrales Zwischenwirbelimplantat, welches eine dem Querschnitt eines Wirbelkörper angenäherte Querschnittsform aufweist, was deutlich aus Figur 10 erkennbar ist. Entsprechend ist die ventrale Seite 2 des Zwischenwirbelimplantats 1 konvex ausgebildet, wohingegen die dorsale Seite 3 eben gestaltet ist. Es ist auch denkbar, daß diese dorsale Seite 3 konkav gekrümmt ist. Zwischen diesen beiden Seiten 2 und 3 sind längliche Durchbrüche 4 und 5 erkennbar, die ein Einwachsen von Binde- und Knochengewebe in das Implantat 1 ermöglichen. Ferner sind zwei Ausnehmungen 6 und 7 erkennbar, in denen Ankerbolzen 8 (Figuren 4 und 5) gelagert sind. Schließlich sind Ausnehmungen 9 und 10 angedeutet, die auf der gegenüberliegenden Anlagefläche 11 aus dem Implantat 1 ausmünden. Die Ausnhemungen 6 und 7 sowie 9 und 10 befinden sich in einer Ebene. Ferner ist erkennbar, daß die Anlagefläche 12 sowie die Anlagefläche 11 (Figur 2) eine profilierte Oberfläche aufweist, die sägezahnartig ausgebildet ist. Dabei wirkt die Sägezahnung 13 der Anlagefläche 12 für das Implantat 1 nach ventral hemmend und die Sägezahnung 14 der Anlagefläche 11 für das Implantat 1 nach dorsal hemmend. Diese Sägezahnungen 13 und 14 verhindern ein Verrutschen des Zwischenwirbelimplantats 1 auf bzw. zwischen den anliegenden Wirbelkörpern 15 (Figur 10).

In der Seitenansicht des Zwischenwirbelimplantats 1, die in der Figur 2 dargestellt ist, ist ein weiterer Durchbruch 16 erkennbar, der ein Einwachsen von Binde- und Knochengewebe erlaubt. Außerdem ist die keilförmige Ausgestaltung erkennbar, wobei der Keilwinkel α zwischen 2° und 15° liegt. Die Dicke des Implantats 1 auf der ventralen Seite 2 beträgt 5 mm und auf der dorsalen Seite 8 mm.

Aus dem in Figur 3 dargestellten Schnitt III-III ist erkennbar, daß zwischen den beiden Durchbrüchen 4 und 5 eine Bohrung 17 liegt, die als Lager für eine insgesamt mit 18 bezeichnete Nockenwelle dient. Diese Nockenwelle 18 bildet das Getriebe für die Ankerbolzen 8, insbesondere für deren Verschiebung von der in der Figur 4 dargestellten Ruhelage in die in der Figur 5 dargestellte Arbeitslage.

Die Nockenwelle 18 weist drei Bünde 19 bis 21 auf, über die sie gelagert ist. Zwischen den Bünden 19 und 20 befinden sich zwei Nocken 22 und 23 und zwischen den Bünden 20 und 21 befinden sich zwei Nocken 24 und 25. Die Bünde 19 bis 21 überragen die Nocken 22 bis 25 radial, so daß zwischen den Oberflächen der Nocken 22 bis 25 und der Bohrungswand 17 stets ein Freiraum verbleibt. Aus Figur 3 ist ferner ein im Bund 21 vorgesehenes Innengewinde 26 angedeutet, in das ein Werkzeug 30, welches einen Gewindebolzen 32 aufweist, eingeschraubt und mit diesem die Nockenwelle 18 gedreht werden kann. Das Werkzeug 30 kann eine nicht gezeigte Handhabe zum einfachen Ergreifen aufweisen. Das Innengewinde 26 liegt geringfügig exzentrisch, insbesondere koaxial zur Nocke 25, so daß keine unnötigen Wandschwächungen eintreten.

Nach der Fixierung des Implantats 1 kann die Nockenwelle 18 mit dem Werkzeug 30 entfernt und gegen eine die Ankerbolzen 8 in der Arbeitslage haltende Einrichtung 29 ersetzt werden. Diese in der Figur 3a dargestellte Einrichtung 29 weist ebenfalls ein Gewinde 26 auf und ist mit einem minimalen radialen Vorsprung 31 versehen (in der Zeichnung übertrieben dargestellt), der eine Verklemmung des Einsatzes 29 im Implantat 1 gewährleistet. Die Nockenwelle 18 und das Werkzeug 30 bilden also ein Instrument, weshalb die Nockenwelle 18 mehrfach verwendbar ist.

In Figur 4 ist die Nockenwelle 18 geschnitten dargestellt und es sind die Ankerbolzen 8 in ihrer Ruhelage, d.h. in ihrer eingefahrenen Stellung gezeigt. Dabei liegen die proximalen Enden 27 auf den Nocken 22 bis 25 auf und die distalen Enden 28 überragen die Anlageflächen 11 und 12 geringfügig, d.h. nur soweit, daß ein Einschieben des Zwischenwirbelimplantats 1 zwischen zwei Wirbelkörper 15 problemlos möglich ist.

In Figur 5 sind die Ankerbolzen 8 in ausgefahrener Stellung dargestellt und die Nockenwelle 18 nimmt eine um 180° gedrehte Lage ein. Ferner ist erkennbar, daß die proximalen Enden 27 der Ankerbolzen 8 bündig zur Bohrungswand 17 liegen.

Die radial versetzte Lage des Innengewindes 26 ist auch deutlich aus Figur 6 erkennbar, die eine Stirnansicht der Nockenwelle 18 der Figur 4 zeigt. Ein Schnitt VII-VII gemäß Figur 5 durch die Nockenwelle 18 ist in Figur 7 dargestellt, wo die beiden Nocken 24 und 25 erkennbar sind. Bei dieser Ausführungsform der Nockenwelle wird der Hub der Nocke durch eine Drehung um 180° erreicht, so daß sich der Stellweg über 180° erstreckt. Ebenfalls aus Figur 7 ist erkennbar, daß die beiden Nocken 24 und 25 einen Winkelversatz von 180° aufweisen, so daß einander gegenüberliegende und die beiden Anlageflächen 11 und 12 durchgreifende Ankerbolzen 8 gleichzeitig ausgeschoben werden.
Bei dem in der Figur 8 dargestellten Ausführungsbeispiel einer Nockenwelle 18 erstreckt sich der Stellweg der Nocke ebenfalls über 180°, wobei aber zum Ausfahren von einander gegenüberliegenden Ankerbolzen 8 lediglich eine einzige Nocke erforderlich ist, so daß die doppelte Anzahl von Ankerbolzen 8 bewegt werden kann. Diese Nocke hat eine vorgegebene Drehrichtung. Die Nockenwelle weist deshalb auch einen Drehanschlag, der in der Zeichnung nicht dargestellte ist, auf.

In der Figur 9 ist eine weitere Ausführungsform einer Nocke dargestellt, die einen sich über 360° erstreckenden Stellweg besitzt. Mit derart gestalteten Nocken können lange Verschiebewege bei geringen Verschiebekräften erzielt werden.

Auch diese Nocke weist eine definierte Drehrichtung und somit die Nockenwelle einen Drehanschlag auf.

## Patentansprüche

1. Ventrales Zwischenwirbelimplantat zum Einsatz z.B. zwischen zwei Wirbelkörper (15) einer Wirbelsäule mit einer oberen und einer unteren Anlagefläche (11 und 12) und wenigstens einem wenigstens eine der Anlageflächen (11 bzw. 12) überragenden Ankerbolzen (8), wobei der wenigstens eine Ankerbolzen (8) versenkbar angeordnet ist und das Implantat (1) zum Ausfahren des Ankerbolzens (8) ein Getriebe mit einer drehbar gelagerten Welle (18) aufweist, wobei das Getriebe ein Instrument oder ein Teil eines Instruments ist, dadurch gekennzeichnet, daß die Welle (18) nach Art einer Nockenwelle mit einer Nocke oder mehreren axial hintereinander angeordneten Nocken (22 bis 25) ausgebildet ist.

2. Zwischenwirbelimplantat nach Anspruch 1, dadurch gekennzeichnet, daß, zwischen den Nocken (22 bis 25) oder zwischen mehreren Nocken (22 bis 25) jeweils ein die Nocken (22 bis 25) radial überragender, scheibenförmiger Bund (19 bis 21) vorgesehen ist, der insbesondere die Lagerstelle der Welle (18) bildet.

3. Zwischenwirbelimplantat nach einem der Ansprüche 1 oder 2, dadurch gekennzeichnet, daß die Welle (18) in einer zwischen den Anlageflächen (11 und 12) vorgesehenen Bohrung (17) gelagert ist und die Welle (18) insbesondere ventral zugänglich ist, wobei die Welle (18) insbesondere über ein Werkzeug drehbar ist.

4. Zwischenwirbelimplantat nach einem der vorhergehenden Ansprüche, dadurch gekennzeichnet, daß eine Stirnseite der Welle (18) ein Innengewinde (26) oder eine entsprechende Werkzeugangriffseinrichtung aufweist.

5. Zwischenwirbelimplantat nach einem der vorhergehenden Ansprüche, dadurch gekennzeichnet, daß sich der Stellweg der Nocke (22 bis 25) über 90°, 180° oder 360° erstreckt, und insbesondere eine Nocke (22 bis 25) mit zwei sich über jeweils 180° erstreckende Stellwege versehen ist.

6. Zwischenwirbelimplantat nach einem der vorhergehenden Ansprüche, dadurch gekennzeichnet, daß die Welle (17) einen Drehanschlag aufweist.

7. Zwischenwirbelimplantat nach einem der vorhergehenden Ansprüche, dadurch gekennzeichnet, daß die Ankerbolzen (8) einen zylindrischen Querschnitt aufweisen und in entsprechenden Ausnehmungen (6 und 7, 9 und 10) im Implantat (1) gelagert sind.

8. Zwischenwirbelimplantat nach einem der vorhergehenden Ansprüche, dadurch gekennzeichnet, daß das Implantat (1) keilförmig ausgebildet ist und die beiden Anlageflächen (11 und 12) einen Keilwinkel (α) von 2° bis 15°, insbesondere 3° bis 10° einschließen, und insbesondere eine Dicke von 5 mm bis 14 mm, bei Keilform eine Dicke von 5 mm bis 8 mm bzw. 8 mm bis 14 mm aufweist.

9. Zwischenwirbelimplantat nach einem der vorhergehenden Ansprüche, dadurch gekennzeichnet, daß das Getriebe an ein stabförmiges Werkzeug anschraubbar ist.

10. Zwischenwirbelimplantat nach einem der vorhergehenden Ansprüche, dadurch gekennzeichnet, daß das Getriebe dorsal oder ventral in das Implantat einsetzbar ist.

11. Zwischenwirbelimplantat nach einem der vorhergehenden Ansprüche, dadurch gekennzeichnet, daß das Implantat die Ankerbolzen (8) in der Arbeitslage haltende Einrichtungen aufweist.

12. Zwischenwirbelimplantat nach Anspruch 11, dadurch gekennzeichnet, daß die Einrichtungen zum Einfahren der Ankerbolzen (8) lösbar sind.

13. Zwischenwirbelimplantat nach einem der vorhergehenden Ansprüche, dadurch gekennzeichnet, daß zwischen dem Getriebe und der Instrumentenstange ein Kardangelenk oder eine biegsame Welle einsetzbar ist.

## Claims

1. A ventral intervertebral implant for use between two vertebral bodies (15) of a spinal column, for instance, having an upper and lower contact surface (11 and 12) and at least one anchor pin (8) projecting beyond at least one of the contact surfaces (11 or 12), wherein the at least one anchor pin (8) is retractably disposed, and the implant (1) has a gear with a rotatable shaft (18) for extending the anchor pin (8), wherein the gear is an instrument or a part of an instrument, characterized in that, the shaft (18) is designed as a camshaft having a cam or a plurality of cams (22 to 25) arranged axially one after the other.

2. The intervertebral implant as defined by claim 1, characterized in that respectively one disk-shaped collar (19 through 21) that projects beyond the cams (22 through 25) is provided between the cams (22 through 25) or between a plurality of cams (22 through 25), which forms the bearing of the shaft (18).

3. The intervertebral implant as defined by claim 1 or 2, characterized in that the shaft (18) is seated in a bore (17) provided between the contact surfaces (11 and 12) and the shaft (18) is ventrally accessible, wherein the shaft (18) can be rotated by means of a tool.

4. The intervertebral implant as defined by one of the preceding claims, characterized in that one face end of the shaft (18) has an interior thread (26) or a corresponding tool-application device.

5. The intervertebral implant as defined by one of the preceding claims, characterized in that the regulating distance of the cam (22 through 25) extends over 90°, 180°, or 360°, and particularly the cam (22 through 25) is provided with two regulating distances respectively extending over 180°.

6. The intervertebral implant as defined by one of the preceding claims, characterized in that the shaft (17) has a rotational stop.

7. The intervertebral implant as defined by one of the preceding claims, characterized in that the anchor pins (8) have a cylindrical cross-section and are seated in corresponding recesses (6 and 7, 9 and 10) in the implant (1).

8. The intervertebral implant as defined by one of the preceding claims, characterized in that the implant (1) is embodied to be wedge-shaped, and the two contact surfaces (11 and 12) form a wedge angle (a) of 2° to 15°, particularly 3° to 10° and particularly that the implant (1) has a thickness of 5 mm to 14 mm, and, with a wedge shape, a thickness of 5 mm to 8 mm or 8 mm to 14 mm.

9. The intervertebral implant as defined by one of the preceding claims, characterized in that the gear can be threaded on a rod-shaped tool.

10. The intervertebral implant as defined by one of the preceding claims, characterized in that the gear can be inserted dorsally or ventrally into the implant.

11. The intervertebral implant as defined by one of the preceding claims, characterized in that the implant has devices which maintain the anchor bolts (8) in the work position.

12. The intervertebral implant as defined by claim 23, characterized in that the devices for retracting the anchor bolts (8) are releasable.

13. The intervertebral implant as defined by one of the foregoing claims, characterized in that a cardan joint or a flexible shaft can be inserted between the gear and the instrument rod.

## Revendications

1. Implant intervertébral ventral destiné à être inséré par exemple entre deux corps de vertèbres (15) d'une colonne vertébrale, avec une face d'appui supérieure et une face d'appui inférieure (11 et 12) et au moins un axe d'ancrage (8) dépassant d'au moins une des faces d'appui (11 ou 12), le ou lesdits axe(s) d'ancrage (8) étant escamotable(s) et l'implant (1) présentant un mécanisme à axe rotatif (18) pour faire sortir l'axe d'ancrage (8), le mécanisme consistant en un instrument ou une partie d'instrument, caractérisé en ce que l'axe (18) se présente sous la forme d'un arbre à came(s) comportant une came ou plusieurs cames (22 à 25) agencées axialement les unes derrière les autres.

2. Implant intervertébral selon la revendication 1, caractérisé en ce qu'entre les cames (22 à 25) ou entre plusieurs cames (22 à 25) est prévu à chaque fois un collet (19 à 21) en forme de disque et dépassant radialement des cames (22 à 25), ledit collet formant en particulier l'élément de pivotement de l'axe (18).

3. Implant intervertébral selon l'une des revendications 1 à 2, caractérisé en ce que l'axe (18) est logé dans un alésage (17) prévu entre les faces d'appui (11 et 12) et en ce que l'axe (18) est accessible en particulier par voie ventrale, l'axe (18) pouvant en particulier être entraîné en rotation au moyen d'un outil.

4. Inplant intervertébral selon l'une des revendications précédentes, caractérisé en ce qu'une extrémité frontale de l'axe (18) présente un taraudage (26) ou un agencement correspondant formant empreinte pour l'outil.

5. Implant intervertébral selon l'une des revendications précédentes, caractérisé en ce que la course de la came (22 à 25) est de 90°, 180° ou 360°, et en ce qu'en particulier une came (22 à 25) présente deux courses de 180°.

6. Implant intervertébral selon l'une des revendications précédentes, caractérisé en ce que l'axe (18) présente une butée en rotation.

7. Implant intervertébral selon l'une des revendications précédentes, caractérisé en ce que les axes d'ancrage (8) présentent une section transversale cylindrique et sont logés dans des évidements correspondants (6 et 7, 9 et 10) que présente l'implant (1).

8. Implant intervertébral selon l'une des revendications précédentes, caractérisé en ce que l'implant (1) affecte la forme d'un cône, et en ce que les deux faces d'appui (11 et 12) présentent un angle de conicité (α) compris entre 2 et 15°, et en particulier entre 3 et 10°, et ont une épaisseur de 5 à 14 mm, soit, dans le cas de la forme conique, une épaisseur de 5 à 8 mm ou de 8 à 14 mm.

9. Implant intervertébral selon l'une des revendications précédentes, caractérisé en ce que le mécanisme peut être vissé sur un outil en forme de tige.

10. Implant intervertébral selon l'une des revendications précédentes, caractérisé en ce que le mécanisme peut être inséré dans l'implant par voie dorsale ou ventrale.

11. Implant intervertébral selon l'une des revendications précédentes, caractérisé en ce que l'implant présente des dispositifs maintenant les axes d'ancrage (8) en position de travail.

12. Implant intervertébral selon la revendications 11, caractérisé en ce que les dispositifs d'engagement des axes d'ancrage (8) sont amovibles.

13. Implant intervertébral selon l'une des revendications précédentes, caractérisé en ce qu'un cardan ou un axe flexible peut être inséré entre le mécanisme et la tige de l'instrument.
